# EUROPÄISCHE PATENTSCHRIFT

(11) **EP 2 646 066 B1**
(45) Veröffentlichungstag und Bekanntmachung des Hinweises auf die Patenterteilung: **14.03.2018**
(21) Anmeldenummer: 11824298.1
(22) Anmeldetag: 05.12.2011
(51) Int. Cl.: A61L 29/08, A61L 29/16

(54) **BESCHICHTUNG UND BESCHICHTUNGSVERFAHREN FÜR DEN BALLON EINES BALLONKATHETERS SOWIE BALLONKATHETER MIT BESCHICHTETEM BALLON**
COATING AND COATING METHOD FOR THE BALLOON OF A BALLOON CATHETER, AND BALLOON CATHETER WITH COATED BALLOON
REVÊTEMENT ET PROCÉDÉ DE REVÊTEMENT D'UN BALLONNET D'UN CATHÉTER À BALLONNET ET CATHÉTER À BALLONNET ENDUIT

(30) Priorität: 04.12.2010 DE 202010016123 U; 26.01.2011 DE 102011000340; 14.06.2011 DE 102011051059
(43) Veröffentlichungstag der Anmeldung: 09.10.2013
(73) Patentinhaber: Aachen Scientific International PTE. LTD., 079903 Singapore (SG)
(72) Erfinder: Rübben Alexander, 98000 Monaco (MC)
(74) Vertreter: Schöneborn, Holger
(86) Internationale Anmeldenummer: PCT/DE2011/075300
(87) Internationale Veröffentlichungsnummer: WO 2012/072074

(56) Entgegenhaltungen:
- WO-A1-2010/136604
- DE-A1-102007 003 184
- US-A1- 2007 065 481
- US-A1- 2008 021 385
- US-A1- 2010 209 471

## Beschreibung

### TECHNISCHES GEBIET DER ERFINDUNG

Die Erfindung betrifft eine Beschichtung für den Ballon eines Ballonkatheters, einen Ballon eines Ballonkatheters mit der Beschichtung sowie einen Ballonkatheter mit einem solchen Ballon. Die Erfindung betrifft ferner ein Verfahren zur Beschichtung eines wirkstoffbeschichteten Ballons, wie in den Ansprüchen erläutert.

### HINTERGRUND DER ERFINDUNG

Die sogenannten "minimal invasiven Verfahren" in der Medizin bieten Kardiologen und Radiologen Möglichkeiten zur Behandlung oder Diagnose von Gefäßen, können aber das Risiko einer Verdickung der Gefäßwand mit konsekutiver Lumeneinengung durch eine Zellproliferation bergen. Um diesem Risiko zu begegnen, sind Wirkstoffbeschichtungen zur Medikamentenabgabe von der Oberfläche des Ballons eines Ballonkatheters bekannt.

Die WO 2008/061642 A2 zeigt Verfahren, bei denen die Oberfläche eines Ballons eines Ballonkatheters chemisch oder durch Aufrauhen strukturiert beziehungsweise profiliert wird, um eine Wirkstoffbeschichtung auf dem Ballon zu verbessern.

Die WO 2009/124570 A1 zeigt ein Verfahren, bei dem ein Wirkstoff durch Anweichen der Oberfläche eines Ballons eines Ballonkatheters in die Oberfläche des Ballons eingebettet wird.

Die WO 2010/009904 A2 zeigt ein Verfahren, bei dem eine Wirkstoffbeschichtung auf der Oberfläche eines Ballons eines Ballonkatheters versprödet wird.

Zur Handhabung lipophiler Wirkstoffe, wie z.B. Paclitaxel, sind Lösungsvermittler bekannt. wie z.B. Phosphatidylcholin, polyethoxyliertes Rizinusöl oder ein Gemisch von Cardiolipin, Phosphatidylcholin und Cholesterol, mit denen Wirkstoffe in durch Lösungsvermittler in nach außen hydrophile Mizellen eingeschlossen werden können. Die EP 2253 337 A1 zeigt Wirkstoffkapseln in Polymerbeschichtungen von Katheterballons. Die WO 2010/136604 A1 zeigt Wirkstoffmizellen und, als Alternative, mittels Tensidlösungsvermittlern suspendierte Wirkstoffpartikel, die in Polymerbeschichtungen eingebracht werden. Die WO 00/32267 A2 und die US 2002/0123505 A1 zeigen Polymerbeschichtungen als Wirkstoffträger.

Zur Handhabung lipophiler Wirkstoffe bei der Einbettung in die Oberfläche eines Ballons eines Ballonkatheters lehrt die WO 2004/028610 A2 eine niedermolekulare Matrixsubstanz aus Zuckern mit einem Molekulargewicht kleiner 5000 Da, und die WO 2000/121840 A2 zeigt eine Schellackbeschichtung mit Paclitaxel. Die EP 1669092 A1 zeigt mit abgebautem Dextran beschichteten kolloidalen Magnetit als Träger für einen antihyperplastischen Wirkstoff.

### OFFENBARUNG DER ERFINDUNG

Der Erfindung liegt die Aufgabe zugrunde, modifizierte Wirkstoffbeschichtungen mit lipophilen Wirkstoffen der Ballons von Ballonkathetern, insbesondere mit einer verzögerten bzw. langfristigen Wirkstoffabgabe nach Aufblasen und Anpressen eines Ballons an das den Ballon umgebende Gefäßgewebe, insbesondere mit geringem Aufwand für Reproduzierbarkeit, Qualitätssicherung und/oder Herstellung, zu ermöglichen. Vorzugsweise soll ermöglicht werden, den Wirkstoff so abzugeben, daß 48 Stunden nach Dilatation des Ballons noch Wirkstoffkonzentrationen in dem Gefäßgewebe vorliegen, das den Ballon bei der Dilatation umgeben hat, welche noch 1/2 bis 1/10, vorzugsweise 1/3 bis 1/5 der Wirkstoffkonzentrationen entsprechen, die zwei Stunden nach Dilatation des Ballons in dem Gefäßgewebe vorlagen, das den Ballon bei der Dilatation umgeben hat. Weiter bevorzugt soll ermöglicht werden, den Wirkstoff derart langfristig abzugeben, daß mehrere Tage nach Dilatation des Ballons noch signifikante Wirkstoffkonzentrationen in demjenigen Gefäßgewebe vorliegen, das den Ballon bei der Dilatation umgeben hat. Die Erfindung soll auch einen entsprechend verbesserten Ballon für einen Ballonkatheter sowie einen Ballonkatheter mit einem entsprechend verbesserten Ballon angeben, mittels welchem die Wirkstoffabgabe im Hinblick auf die o.g. verzögerte bzw. langfristige Abgabe positiv beeinflußt werden kann.
Unter einem weiteren Aspekt soll ermöglicht werden, den Wirkstoff so abzugeben, daß Wochen nach Dilatation des Ballons noch Wirkstoffkonzentrationen in dem Gefäßgewebe vorliegen.

Unter einem Aspekt der Erfindung ermöglicht die modifizierte Wirkstoffbeschichtung eine günstige Pharmakokinetik, gemäß der in dem Gewebe, das einen Ballon bei dessen Expansion umgeben hat, langfristig erhöhte und wirksame Wirkstoffkonzentrationen vorliegen.

Die oben genannte Aufgabe wird gelöst von einer lösungsvermittlerfreien Beschichtung für einen Ballon eines Ballonkatheters, welche wenigstens einen lipophilen Wirkstoff und ein Mittel zur Modifizierung der Wirkstoffabgabe an ein den Ballon umgebendes Gefäß umfaßt, wobei das Mittel zur Modifizierung der Wirkstoffabgabe ein Polysaccharid (polymeres Kohlenhydrat) mit einer mittleren Molekularmasse von 10.000 Da bis 100.000.000 Da, insbesondere 20.000 Da bis 500.000 Da gemäss Anspruch 1 ist. Das Polysaccharid ist bevorzugt ein verzweigtes Polysaccharid. Eine lösungsvermittlerfreie Beschichtung ist dabei eine Beschichtung, die ohne Zusatz von Lösungsvermittlern hergestellt ist. Natürliches Dextran wird als Polysaccharid verwendet. Natürliche Dextrane werden industriell im allgemeinen enzymatisch durch Bakterien oder durch Hefen aus Saccharose hergestellt und besitzen mittlere Molekularmassen zwischen 10.000 und 50.000.000 Da.
Bei einer bevorzugten Durchführungsform des erfindungsgemäßen Verfahrens, bei dem Dextran als Polysaccharid verwendet wird, hat das Dextran eine Molekularmasse zwischen 20.000 und 80.000 Da. Besonders bevorzugt wird dabei Dextran 40 verwendet, ein Dextran mit einer mittleren Molekularmasse von etwa 40.000 Da.
Es wird vermutet, daß nach einer Gefäßdilatation durch einen Ballon mit einer erfindungsgemäßen Beschichtung, insbesondere mit einer erfindungsgemäßen dextranhaltigen Beschichtung, selbst nach Entnahme des Ballons an der Gefäßwand eine Polysaccharid- bzw. Dextranmatrix mit dem Wirkstoff haftet und eine Abgabe des Wirkstoffs aus dieser Matrix an das Gewebe des Gefäßes erfolgt, wobei die Abgabe durch diese Matrix verzögert bzw. langfristig erfolgt. Dabei ermöglicht insbesondere die Verwendung von Dextran mit einer mittleren Molekularmasse von 20.000 Da bis 80.000 Da, vorteilhaft 40.000 Da bis 80.000 Da und besonders bevorzugt von Dextran 40, eine langfristige Wirkstoffabgabe. So konnte durch Tierversuche am Schwein nachgewiesen werden, daß mit der Erfindung eine langfristige Wirkstofffreigabe derart erzielt werden konnten, daß 48 Stunden nach Dilatation des Ballons in dem Gefäßgewebe, das bei der Dilatation den Ballon umgeben hat, noch Wirkstoffkonzentrationen vorlagen, welche 1/2 bis 1/10, insbesondere 1/3 bis 1/5 der Wirkstoffkonzentration entsprachen, die zwei Stunden nach Dilatation des Ballons vorlagen.

Eine erfindungsgemäße Beschichtung kann durch zumindest teilweises Benetzen der Oberfläche eines mit einem lipophilen Wirkstoff beschichteten Ballons eines Ballonkatheters mit einer Lösung hergestellt werden. Diese Lösung besteht aus wenigstens einem Polysaccharid und wenigstens einem Lösungsmittel. Wird dann das Lösungsmittel von dem benetzten Ballon entfernt, so verbleibt auf dem Ballon eine Beschichtung gemäß der Erfindung.

Bei einer erfindungsgemäßen Beschichtung für einen Ballon eines Ballonkatheters mit einer Schicht eines lipophilen Wirkstoffs und wenigstens einem Mittel zur Modifizierung der Wirkstoffabgabe an ein den Ballon umgebendes Gefäß, wobei das Mittel zur Modifizierung der Wirkstoffabgabe ein Polysaccharid mit einer mittleren Molekularmasse von 10.000 Da bis 100.000.000 Da ist, liegt das Mittel zur Modifizierung der Wirkstoffabgabe in Form einer Schicht vor, die direkt oder indirekt auf der dem Ballon abgewandten Seite der Wirkstoffschicht vorgesehen ist, was ebenfalls die oben genannte Aufgabe löst. Diese Beschichtung kann alle genannten Aspekte der lösungsvermittlerfreien Beschichtung aufweisen.

Bei einem erfindungsgemäßen Ballon eines Ballonkatheters kann der lipophile Wirkstoff vorteilhaft direkt auf der Oberfläche des Ballons angeordnet sein und das Polysaccharid den Wirkstoff und den Ballon bedecken. Der Ballon kann so unter Verwendung von herkömmlichen Standardballons mit Wirkstoffbeschichtung leicht insbesondere dadurch hergestellt werden,
- daß die wirkstoffbeschichtete Oberfläche des Ballons zumindest teilweise, vorzugsweise vollständig mit einer wenigstens ein oben genanntes Polysaccharid und wenigstens ein Lösungsmittel enthaltenden Lösung benetzt wird und
- daß das Lösungsmittel von dem Polysaccharid getrennt wird.

Zur Aufbringung des Wirkstoffs kann die Oberfläche des Ballons, insbesondere eine Polymeroberfläche, zumindest teilweise mit einer ersten Lösung eines Wirkstoffes benetzt werden, insbesondere von Paclitaxel in einem organischen Lösungsmittel, beispielsweise einer konventionellen Paclitaxel-Methylenchlorid-Lösung.
Anschließend kann zumindest der mit der ersten Lösung eines Wirkstoffes benetzte Teil der Oberfläche des Ballons erfindungsgemäß mit einer zweiten Lösung mit dem oben genannten Polysaccharid, d.h. Dextranlösung benetzt werden, bei welcher ein Wasser-Alkoholgemisch als Lösungsmittel dient. Diese Lösung hat vorzugsweise einen Gehalt des Polysaccharids von 1 - 15 Gew.-%, weiter bevorzugt 2 bis 10 Gew.-%, weiter bevorzugt 3 - 8 Gew.-%, insbesondere 5 Gew.-%, wobei ein bevorzugtes Polysaccharid Dextran 40 ist. Das Wasser-Alkoholgemisch weist bevorzugt einen Alkoholgehalt von 25 - 50 Gew.-%, weiter bevorzugt 30 bis 45 Gew.-%, weiter bevorzugt 35 - 42 Gew.-%, insbesondere 39 Gew.-% auf, wobei der Alkoholgehalt vorzugsweise durch Ethanol gebildet ist.
Die mit der ersten Lösung eines Wirkstoffes benetzte Oberfläche des Ballons wird vorzugsweise schnell getrocknet, beispielsweise in 2 bis 7 Minuten.
Durch das Benetzen der Oberfläche des Ballons mit der ersten Lösung eines lipophilen Wirkstoffes und Trocknen wird auf der Oberfläche eine lackartige, transparente Wirkstoffschicht erzeugt. Diese Benetzung hat sich als Basis für eine homogene und reproduzierbare Wirkstoffbeladung erwiesen und dient vorteilhaft dem Aufbringen der gesamten Wirkstoffbeladung.

Durch das Benetzen mit der zweiten Lösung, vorzugsweise einer wäßrigen Dextran-Alkohol-Lösung wird die gesamte Beschichtung spröder und optisch weniger transparent, also milchiger. Die so erzeugte Oberfläche hat eine kreideartige, Konsistenz. Es wird vermutet, dass bei der erfindungsgemäßen Beschichtungsausbildung in der zweiten Lösung der Alkohol die Versprödung des Wirkstoffes ermöglicht und sich insbesondere Dextran-Moleküle zwischen den Molekülen des Wirkstoffes, insbesondere Paclitaxel ablagern können, um eine homogene feine Verteilung des Wirkstoffs in der Polysaccharid-WirkstoffBeschichtung und dadurch eine besonders lange Wirkstoffabgabe an das umliegende Gewebe im Körper zu erreichen.
Die Langzeitfreisetzung ergibt vorzugsweise über 1 - 3 Wochen, insbesondere 1 - 2 Wochen nach Dilatation eines Ballons eines Ballonkatheters mit einer erfindungsgemäßen Wirkstoffbeschichtung eine Abgabe wirksamer Dosen des Wirkstoffs an Gewebe, das bei der Dilatation den Ballon umgab.
Grundsätzlich kann sowohl die gesamte Ballonoberfläche, als auch nur ein Teil der Ballonoberfläche, beispielsweise der beim Aufdehnen mit dem Gewebe in Kontakt kommende Bereich der Oberfläche, mit dem erfindungsgemäßen Verfahren beschichtet werden.
Die Erfindung betrifft auch einen Ballonkatheter mit einem erfindungsgemäßen Ballon. Ballons, die mit einer erfindungsgemäßen Beschichtung versehen werden können bzw. einem erfindungsgemäßen Verfahren unterzogen werden können, sind z.B. in den Druckschriften WO 2008/061642 A2, WO 2009/124570 A1 und WO 2010/009904 A2 gezeigt. Ein Mittel zur Modifizierung der Abgabe eines auf eine Oberfläche eines Ballons eines Ballonkatheters aufgebrachten lipophilen Wirkstoffs kann dadurch ausgebildet werden,
- daß die Oberfläche eines Ballons eines Ballonkatheters zumindest teilweise mit einer wenigstens ein oben genanntes Polysaccharid und wenigstens ein Lösungsmittel enthaltenden Lösung benetzt wird und
- daß das Lösungsmittel von dem Polysaccharid getrennt wird.

Das Polysaccharid ist ein natürliches Dextran, insbesondere Dextran mit einer mittleren Molekularmasse zwischen 20.000 und 80.000 Da, vorteilhaft 40000 - 80000 Da. Dabei kann der Wirkstoff auf der Oberfläche des Ballons eine Struktur mit Vertiefungen bilden, wobei die Benetzung mit der Lösung und die Trennung von Lösungsmittel und Polysaccharid derart erfolgen, daß das Polysaccharid zumindest partiell in die Vertiefungen eindringt.
Das Lösungsmittel kann von dem Polysaccharid durch Trocknung getrennt werden.

Das Lösungsmittel kann Wasser oder ein wäßriges Lösungsmittelgemisch sein. Ein solches Lösungsmittelgemisch enthält vorzugsweise eine leicht flüchtige organische Komponente, insbesondere einen Alkohol, insbesondere Ethanol. Die Trocknung kann mit einer organischen Komponente wie Ethanol im Vergleich zu reinem Wasser als Lösungsmittel beschleunigt werden. Die organische Komponente ist vorteilhaft ein Alkohol, ein Keton oder eine andere organische Verbindung, die einen höheren Dampfdruck als Wasser aufweist und mit Wasser mischbar ist. Als Beispiele können Methanol, Ethanol, Isopropanol und Aceton genannt werden. Eine polare protische Lösungsmittelkomponente wie vorzugsweise Wasser ist vorteilhaft für die Lösung.
Die Lösung, mit der der Ballon benetzt wird, kann eine gesättigte Lösung sein.
Die Benetzung des Ballons geschieht vorzugsweise durch Eintauchen des zumindest teilweise aufgeblasenen Ballons in die Lösung.
Eine Trennung des Lösungsmittels durch Trocknung kann bevorzugt durch Verflüchtigenlassen an der Luft erfolgen. Maßnahmen wie Wärmezufuhr oder Erzeugen von Unterdruck sind dabei zur Ausbildung einer vorteilhaften Beschichtung nicht notwendig.
Der Ballon kann nach dem Entfernen des Lösungsmittels gefaltet oder zusammengerollt werden.

Als Wirkstoff können bei der Erfindung beispielsweise Tretinoin und/oder Tretinoinderivate und/oder Orphanrezeptoragonisten und/oder Elafinderivate und/oder Corticosteroide und/oder Steroidhormone und/oder Paclitaxel und/oder Sirolimus und/oder Paclitaxelderivate und/oder Rapamune und/oder Tacrolimus und/oder Proteine und/oder Peptide und/oder zellproliferationsverändernde Substanzen verwendet werden. Als Steroidhormone können insbesondere Methylprednisolon, Dexamethason oder Östradiol verwendet werden. Vorteilhaft werden allgemein zellproliferationsverändernde Substanzen als Wirkstoff verwendet.

### BESCHREIBUNG BEVORZUGTER AUSFÜHRUNGSFORMEN

### Beispiel 1:

Eine Dextranlösung mit 5 Gew.-% Dextran40 wurde hergestellt, wobei das Lösungsmittel ein Wasser-Ethanol-Gemisch mit 39 Gew.-% Ethanol war.

Es wurde ein mit einer Paclitaxelschicht versehener Ballon vom Typ ELUTAX 16350 der Aachen Resonance GmbH, D-52074 Aachen mit Abmessungen von 3,5 mm Durchmesser und 16 mm Länge und einer Paclitaxelbeschichtung mit einer Beladungskonzentration von 2 Microgramm/mm² verwendet. Der Ballon wurde in die Dextranlösung getaucht und mit einer Geschwindigkeit von 1 cm/Sekunde über seine Länge von 16 mm herausgezogen.

Nachdem der Ballon vollständig aus der Lösung herausgezogen war, wurde der Ballonkatheter an der Luft getrocknet.

### Beispiel 2:

Im Tierversuch am Schwein wurde unter Betäubung an einem Tier jeweils in zwei vergleichbaren Gefäßen einmal eine Dilatation mit einem Ballon vom Typ ELUTAX 16350 und einmal eine Dilatation mit dem nach obigem Beispiel 1 erhaltenen Ballon vorgenommen. Das Tier wurde nach zwei Tagen getötet, worauf die den Dilatationen unterzogenen Bereiche der Gefäße entnommen und kurz gespült wurden, um die Konzentration von Paclitaxel pro mg der entnommenen Gefäßproben 48 Stunden nach den Dilatationen zu bestimmen. 48 Stunden nach der jeweiligen Dilatation zeigte der mit dem Ballon des obigen Beispiels 1 dilatierte Gefäßbereich im Vergleich zu dem mit dem Ballon vom Typ ELUTAX 16350 dilatierten Gefäßbereich eine mehr als 100-fach höhere Paclitaxelkonzentration im Gewebe.

Dieses Ergebnis zeigt die Wirkung der Dextranschicht für die langfristige bzw. 5 verzögerte Abgabe des Wirkstoffs Paclitaxel.

## Patentansprüche

1. Ballon für einen Ballonkatheter mit einer Beschichtung, welche eine Schicht eines lipophilen Wirkstoff und wenigstens ein Mittel zur Modifizierung der Wirkstoffabgabe an ein den Ballon umgebendes Gefäß umfasst, wobei das Mittel zur Modifizierung der Wirkstoffabgabe ein Polysaccharid mit einer mittleren Molekularmasse von 10.000 Da bis 100.000.000 Da ist,
**dadurch gekennzeichnet,**
**dass** der Wirkstoff auf der Oberfläche des Ballons angeordnet ist und das Polysaccharid in Form einer Schicht vorliegt, die auf der dem Ballon abgewandten Seite der Schicht des Wirkstoffs aufgebracht ist und den Wirkstoff bedeckt, wobei das Polysaccharid ein natürliches Dextran ist.

2. Ballon nach Anspruch 1, **dadurch gekennzeichnet, dass** das Dextran eine mittlere Molekularmasse zwischen 20.000 und 80.000 Da hat.

3. Ballonkatheter, umfassend einen Ballon nach Anspruch 1 oder 2.

4. Verfahren zur Beschichtung eines auf einer Oberfläche mit einem lipophilen Wirkstoff beschichteten Ballons eines Ballonkatheters zur Herstellung eines Ballons nach Anspruch 1 oder 2, **dadurch gekennzeichnet, dass**
- die wirkstoffbeschichtete Oberfläche des Ballons zumindest teilweise mit einer wenigstens ein Polysaccharid mit einer mittleren Molekularmasse von 10.000 Da bis 100.000.000 Da und wenigstens ein Lösungsmittel enthaltenden Lösung benetzt wird und
- das Lösungsmittel von dem Polysaccharid getrennt wird,
wobei das Polysaccharid ein natürliches Dextran ist.

5. Verfahren nach Anspruch 4, **dadurch gekennzeichnet, dass** der Wirkstoff auf der Oberfläche des Ballons eine Struktur mit Vertiefungen bildet, wobei die Trennung von Lösungsmittel und Polysaccharid derart erfolgt, dass das Polysaccharid zumindest partiell in die Vertiefungen eindringt.

6. Verfahren nach Anspruch 4 oder 5, **dadurch gekennzeichnet, dass** das Dextran eine mittlere Molekularmasse zwischen 20.000 und 80.000 Da hat.

7. Verfahren nach einem der Ansprüche 4 bis 6, **dadurch gekennzeichnet, dass** das Lösungsmittel von dem Polysaccharid durch Trocknung getrennt wird.

8. Verfahren nach einem der Ansprüche 4 bis 7, **dadurch gekennzeichnet, dass** das Lösungsmittel ein wässriges Lösungsmittelgemisch ist.

## Claims

1. Balloon for a balloon catheter, comprising a coating, which comprises a layer of a lipophilic active substance and at least one agent for modifying the release of active substance to a vessel surrounding the balloon, wherein the agent for modifying the release of active substance is a polysaccharide with a mean molecular mass of 10,000 Da to 100,000 Da,
**characterized in that**
the active substance is arranged on the surface of the balloon and that the polysaccharide is in form of a layer applied on the side of the layer of the active substance that is averted from the balloon and covering the active substance, wherein the polysaccharide is a natural dextran.

2. Balloon according to claim 1, **characterized in that** the natural dextran has a mean molecular mass ranging between 20,000 and 80,000 Da.

3. Balloon catheter, comprising a balloon according to claim 1 or 2.

4. Method for coating a balloon of a balloon catheter, said balloon being coated on a surface with a lipophilic active substance, for manufacturing a balloon according to claim 1 or 2, **characterized in that**
- the active-substance-coated surface of the balloon is moistened at least partly with a solution containing at least one polysaccharide having a mean molecular mass of 10,000 Da to 100,000,000 Da and at least one solvent, and
- the solvent is separated from the polysaccharide,
wherein the polysaccharide is a natural dextran.

5. Method according to claim 4, **characterized in that** the active substance forms a structure with deepenings on the surface of the balloon, wherein the separation of the solvent and the polysaccharide is realized in such a manner that the polysaccharide penetrates at least partly into the deepenings.

6. Method according to claim 4 or 5, **characterized in that** the dextran has a mean molecular mass ranging between 20,000 and 80,000 Da.

7. Method according to any of claims 4 to 6, **characterized in that** the solvent is separated from the polysaccharide by drying.

8. Method according to any of claims 4 to 7, **characterized in that** the solvent is an aqueous solvent mixture.

## Revendications

1. Ballonnet pour un cathéter à ballonnet, présentant un revêtement, qui comprend une couche d'une substance active lipophile et au moins un agent pour la modification de la libération de la substance active à un vaisseau entourant le ballonnet, l'agent pour la modification de la libération de la substance active étant un polysaccharide présentant une masse moléculaire moyenne de 10.000 Da à 100.000.000 Da, **caractérisé en ce que** la substance active est disposée sur la surface du ballonnet et le polysaccharide se trouve sous forme d'une couche qui est appliquée sur le côté de la couche de substance active opposée au ballonnet et qui recouvre la substance active, le polysaccharide étant un dextrane naturel.

2. Ballonnet selon la revendication 1, **caractérisé en ce que** le dextrane présente une masse moléculaire moyenne entre 20.000 et 80.000 Da.

3. Cathéter à ballonnet, comprenant un ballonnet selon la revendication 1 ou 2.

4. Procédé pour le revêtement d'un ballonnet, revêtu sur une surface par une substance active lipophile, d'un cathéter à ballonnet pour la fabrication d'un ballonnet selon la revendication 1 ou 2, **caractérisé en ce que**
- la surface revêtue par une substance active du ballonnet est mouillée au moins partiellement par une solution contenant au moins un polysaccharide présentant une masse moléculaire moyenne de 10.000 Da à 100.000.000 Da et au moins un solvant et
- le solvant est séparé du polysaccharide,
le polysaccharide étant un dextrane naturel.

5. Procédé selon la revendication 4, **caractérisé en ce que** la substance active forme, à la surface du ballonnet, une structure présentant des creux, la séparation du solvant et du polysaccharide ayant lieu de manière telle que le polysaccharide pénètre au moins partiellement dans les creux.

6. Procédé selon la revendication 4 ou 5, **caractérisé en ce que** le dextrane présente une masse moléculaire moyenne entre 20.000 et 80.000 Da.

7. Procédé selon l'une quelconque des revendications 4 à 6, **caractérisé en ce que** le solvant est séparé du polysaccharide par séchage.

8. Procédé selon l'une quelconque des revendications 4 à 7, **caractérisé en ce que** le solvant est un mélange de solvants aqueux.
